(19) Europäisches Patentamt
European Patent Office
Office européen des brevets

(11) Veröffentlichungsnummer: **0 044 976**
**A2**

# (12) EUROPÄISCHE PATENTANMELDUNG

(21) Anmeldenummer: **81105209.1**

(51) Int. Cl.³: **A 61 K 7/42**

(22) Anmeldetag: **04.07.81**

(30) Priorität: **26.07.80 DE 3028417**

(43) Veröffentlichungstag der Anmeldung: **03.02.82**
**Patentblatt 82/5**

(84) Benannte Vertragsstaaten: **CH DE FR GB IT LI**

(71) Anmelder: **BASF Aktiengesellschaft,**
**Carl-Bosch-Strasse 38, D-6700 Ludwigshafen (DE)**

(72) Erfinder: **Thoemel, Frank, Dr. Chem., Leberstrasse 17, D-6940 Weinheim (DE)**
Erfinder: **Hoffmann, Werner, Dr. Chem., Ringstrasse 11C, D-6701 Neuhofen (DE)**
Erfinder: **Degner, Dieter, Dr. Chem., Kurpfalzstrasse 8, D-6701 Dannstadt-Schauernheim (DE)**

(54) **Verwendung von verätherten p-Hydroxyzimtsäureestern als Lichtschutzmittel.**

(57) Verwendung von Verbindungen der allgemeinen Formel I

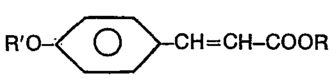

(I)

in der R' einen geradkettigen oder verzweigten Alkylrest mit 3 bis 15 Kohlenstoffatomen, einen Benzyl- oder Phenylrest und R einen geradkettigen oder verzweigten Alkylrest mit 1 bis 15 Kohlenstoffatomen oder den $\beta$-Alkoxyäthan-, 3-Alkoxypropan-, einen Cyclohexyl- oder Benzylrest bedeuten als Lichtschutzmittel oder UV-Stabilisator.

Verwendung von Verbindungen der Formel I als Lichtschutzmittel für die menschliche Haut.

EP 0 044 976 A2

ACTORUM AG

COMPLETE DOCUMENT

# Verwendung von verätherten p-Hydroxyzimtsäureestern als Lichtschutzmittel

Die vorliegende Erfindung betrifft die Verwendung von an der phenolischen 4-Hydroxygruppe verätherten Zimtsäureestern als Lichtschutzmittel, insbesondere für die menschliche Haut oder auf technischem Gebiet als Lichtschutzmittel bzw. UV-Stabilisator.

Es ist bekannt, daß der als UV-B-Strahlung bezeichnete Bereich zwischen 280 und 315 nm des Sonnenlichts oder künstlicher Lichtquellen für die Erythembildung der menschlichen Haut verantwortlich ist. Das Maximum der Wirksamkeit der UV-Strahlung für die Erythembildung liegt bei 297 nm, wenn die Strahlungsintensität für alle Wellenlängen gleich groß ist. Beim Sonnenlicht mit Strahlung unterschiedlicher Intensität ist dieses Maximum auf 308 nm verschoben. Durch geeignete Filtersubstanzen für den UV-B-Bereich gelingt es, die Erythembildung zu verhindern oder zumindest zu verzögern. Die Pigmentbildung der Haut, das heißt die Bräuning, soll daher gewährleistet bleiben.

Die UV-Strahlung ist darüber hinaus auch eine wichtige Einflußgröße bei der Alterung von Polymeren und kann beispielsweise die Umwandlung bestimmter Farbstoffe bewirken, so daß auch für solche Produkte Filtersubstanzen als Stabilisatoren nahezu unentbehrlich sind.

In den vergangenen vierzig Jahren ist eine große Anzahl von chemischen Verbindungen auf ihre Filterwirkung im UV-B-Bereich hin untersucht worden. Ob es sich jedoch bei einer im UV-Gebiet absorbierenden Substanz auch für die menschliche Haut um einen brauchbaren Sonnenschutzfilter handelt, wird noch von anderen Faktoren entscheidend be-

D/BL

stimmt:

Neben der hohen Filterwirksamkeit in dem erythermalen Bereich soll die Substanz eine hohe Durchlässigkeit für die Bräunungsenergie aufweisen, sie sollte eine möglichst ideale Haut- und Schleimhautverträglichkeit besitzen und darf nicht toxisch sein. Sie darf nicht oxidationsempfindlich sein und durch UV-Bestrahlung keine Veränderungen oder Verfärbungen erleiden. Eine entsprechende Zubereitung soll lagerstabil sein, keinen Eigengeruch aufweisen und mit den üblicherweise verwendeten Trägerstoffen oder Verbindungsmittel verträglich sein.

Die bekannten UV-Filter weisen häufig als Nachteile auf, daß sie beim Lagern sowie gegen UV-Strahlung oder sichtbare Strahlung und/oder Luft instabil sind, in gefärbte Zersetzungsprodukte übergehen, die Wäsche anschmutzen oder sogar hautschädlich sein können. Da Sonnenschutzmittel viel von Personen verwendet werden, die im Freien arbeiten, Sport treiben, wie Schwimmen und Tauchen, sollen diese Mittel nicht zu leicht durch Wasser oder Schweiß von der Haut entfernbar sein.

Es gibt nur verhältnismäßig wenig Substanzen, die den angegebenen Ansprüchen mehr oder weniger gut entsprechen, das heißt, daß sich in der Praxis nur relativ wenige Substanzen durchgesetzt haben. Beispielsweise finden verschiedene Ester der p-Methoxyzimtsäure seit langem als Lichtschutzmittel Verwendung (vgl. W. R. Markland Cosmetics and Toiletries 91, 79ff (1976)). Ester von hoheren p-Alkoxyzimtsäuren sind zwar aus Ber. d. dtsch. chem. Ges. 61, S. 2323, bekannt, werden dort aber als Lichtschutzmittel nicht empfohlen.

Es wurde nun gefundem, daß Verbindungen der allgemeinen Formel I,

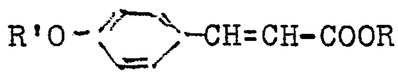 (I)

in der R' einem geradkettigen oder verzweigten Alkylrest mit 3 bis 15 Kohlenstoffatomen, einen Benzyl- oder Phenylrest und R einen geradkettigen oder verzweigten Kohlenwasserstoffrest mit 1 bis 15 Kohlenstoffatomen oder den ß-Alkoxyäthan-, 3-Alkoxypropan-, mit je 1 bis 3 Kohlenstoffatomen im Alkyl, Cyclohexyl- oder Benzylrest bedeuten, als Lichtschutzmittel für die menschliche Haut in kosmetischen Zubereitungen oder als technisches Lichtschutzmittel oder UV-Stabilisator für Kunststoffe, Lösungs- und Anstrichmittel sowie Öle planzlicher oder petrochemischer Herkunft verwendet werden können.

Die Verbindung der Formel I absorbieren mit sehr guter Extinktion im UV-B-Bereich. Durch Variation der Ätherkomponenten für sich oder gleichzeitig mit der Esterkomponenten kann man Lichtschutzmittel mit gezielten Absorptions- und Löslichkeitseigenschaften erhalten. Die Palette der verfügbaren Lichtschutzmittel wird durch die erfindungsgemäß zu verwendenden und chemisch leicht zugänglichen Verbindungen in vorteilhafter Weise bereichert.

Von den für R' und R angegebenen Bedeutungen sind bevorzugt für R' Alkylreste mit 4 bis 10 Kohlenstoffatomen und für R Alkylreste mit 1 bis 8 Kohlenstoffatomen.

Die erfindungsgemäßen Verbindungen werden hergestellt in an sich üblicher Weise durch Kondensation eines entsprechenden verätherten p-Hydroxybenzaldehyd mit einem Essig- oder Malonester, dessen zugrundeliegender Esteralkohol HOR dem Rest R der Formel I entspricht, in Gegenwart von Alkali bei Temperaturen von 0 bis 100°C, wobei bei Verwendung eines Malonesters anschließend partiell verseift und decarboxy-

liert wird.

Beispielhaft sei die Kondensation eines entsprechenden Aldehyds mit einem entsprechenden Essigester zweckmäßigerweise in überschüssiger Menge bei Temperaturen von 50 bis 100°C in Gegenwart von Alkalialkoholat, wie Natriummethylat, angeführt.

Die erfindungsgemäß zu verwendenden Verbindungen finden im Kosmetik-, Farben- und Kunststoffbereich Verwendung. Die neuen Lichtschutzmittel wirken stabilisierend gegenüber Lichteinflüssen in Ölen pflanzlicher und petrochemischer Herkunft sowie Lösungsmitteln. Sie können auch im Gemisch mit anderen Lichtschutzmitteln Verwendung finden. Der Gehalt in den Zubereitungen mit den erfindungsgemäß zu verwendenden Verbindungen hängt weitgehend vom Verwendungszweck dieser Zubereitungen ab und liegt im allgemeinen bei 0,1 bis 15 Gewichtsprozent einer Verbindung der allgemeinen Formel I.

Gegenstand der Erfindung ist demnach auch ein als kosmetisches Mittel oder Zubereitung vorliegendes Lichtschutzmittel mit einem Gehalt von 0,1 bis 15 Gewichtsprozent, bevorzugt 2 bis 8 Gewichtsprozent, einer Verbindung der Formel 1 neben üblichen festen, halbfesten oder flüssigen Trägerstoffen oder Verdünnungsmitteln, Gemischen aus diesen und gegebenenfalls unter Zusatz üblicher kosmetischer Hilfsstoffe.

Von der Art des Trägers oder Verdünnungsmittels hängt es ab, ob das fertige Lichtschutzmittel beispielsweise einer Lösung, ein Öl, eine Creme, eine Salbe, eine Lotion, eine Emulsion oder ein Pulver ist. Derartige Zubereitungen können ~~beispielsweise der~~ Zeitschrift "Fette und Seifen", 53. Jahrgang, Seiten 694-699 (1951), oder der Zeitschrift "Seifen, Öle, Fette, Wachse", 1955, Seite 147, oder

H. Janistyn, Handbuch der Kosmetika und Riechstoffe, Band 3, 1973, Hüthig Verlag Heidelberg, entnommen werden.

Üblicherweise verwendete kosmetische Hilfsstoffe, die als Zusätze in Betracht kommen, sind beispielsweise Emulgatoren, wie Fettalkoholäthoxylate, Sorbitanfettsäureester oder Lanolinderivate, Dickungsmittel, wie Carboxymethylcellulose oder vernetzte Polyacrylsäure, Konservierungsmittel oder Parfüms. Grundlage für Sonnenschutzöle sind beispielsweise pflanzliche Öle, wie Erdnußöl, Olivenöl, Sesamöl, Baumwollsamenöl, Kokosöl, Traubenkernöl, Rizinusöl, oder Mineralöle, wie Vaselinöl, oder insbesondere flüssiges Paraffin, synthetische Fettsäureester und Glyceride.

Grundlage für Seifen sind beispielsweise Vaseline, Lanolin, Eucerin oder Polyäthylenglycole.

Grundlage für Cremes sind beispielsweise fettreiche Cremes, Glyzerin-, Polysaccharid-, Tylosecremes, für Cremes auf Basis von Fetten und Wachsen Cetylalkohol, Lanolincreme, Kakaobutter, Bienenwachs, Stearinsäure, Stearinalkohol, Glyzerinmonostearat, native und mineralische Öle und Fette.

Grundlage für Emulsionen sind beispielsweise Mischungen aus Stearinglykol, einem pflanzlichen und/oder Mineralöl, wie Mandelöl, Paraffinöl und Vaseline und Wasser oder Mischungen aus Äthylalkohol, Wasser, Lanolin und Tragant, oder Mischungen aus Äthylalkohol, Stearin, Wasser oder Tragant, Glyzerin, Alkohol und Wasser oder Mischungen aus Stearinsäure, Paraffinöl, Propyl- oder Isopropylalkohol und Wasser.

Zur Erläuterung des Gegenstandes der Erfindung sind nachstehend einige Beispiele angeführt, die aber keinesfalls einschränkend für die Erfindung anzusehen sind.

Beispiel für die chemische Herstellung

Zu 30 g (0,1 mol) 30proz. Natriummethylat in Methanol gibt man 50 ml Toluol und destilliert in einer Stunde 22 g eines Gemisches aus Toluol und Methanol ab. Zum Rückstand gibt man in einer Stunde bei 60°C ein Gemisch von 31 g (0,15 mol) p-Hexoxybenzaldehyd und 95 g (1,18 mol) Methylacetat und läßt 6 Stunden bei 60°C nachrühren. Nach Zusatz von 30 ml 10proz. Essigsäure extrahiert man mit Toluol, wäscht mit Wasser und destilliert das Lösungsmittel ab. Den Rückstand kristallisiert man aus Äthanol um. Ausbeut 32 g (90 % d.Th.).

Die Verbindungen der folgenden Tabelle werden analog hergestellt:

Tabelle 1

| Verbindungen Nr. | Formel | λ max [nm] | ε (Äthanol) | $E^{1\%}_{1cm}$ (Äthanol) | Schmelzpunkt [°C] Siedepunkt [°C] |
|---|---|---|---|---|---|
| 1 | 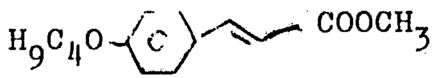 | 310,4 | $2{,}52 \times 10^4$ | 1078 | 77 - 80 |
| 2 | 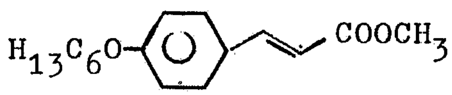 | 310 | $2{,}23 \times 10^4$ | 935 | 83 - 85 |
| 3 | 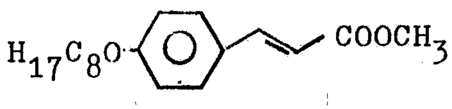 | 310 | $2{,}32 \times 10^4$ | 871 | 70 - 72 |
| 4 | 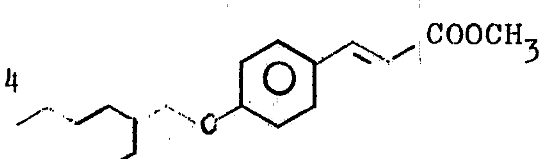 | 310,5 | $2{,}01 \times 10^4$ | 757 | 150 - 152/0,2 mbar |
| 5 | 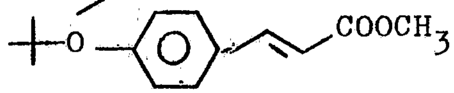 | 293 | $1{,}91 \times 10^4$ | 815 | 50 - 52<br>122 - 124/0,1 mbar |
| 6 | 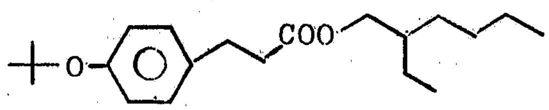 | 294 | $2{,}41 \times 10^4$ | 644 | 170 - 180<br>183 - 185/0,1 mbar |
| 7 | 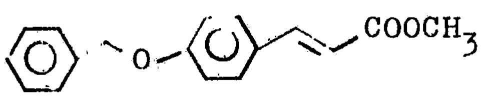 | 308 | $1{,}67 \times 10^4$ | 621 | 118 - 120 |

Beispiele für die Formulierung als Lichtschutzmittel

Die Zahlen in den nachfolgenden Formulierungs-Beispielen sollen Gewichtsteile bedeuten:

Beispiel 1

Sonnenschutzöl

| | |
|---|---|
| 5,5 | 4-(2-Äthylhexoxy)-zimtsäuremethylester |
| 0,2 | p-Hydroxybenzoesäuremethylester |
| 17,8 | Olivenöl |
| 10,0 | Äthylhexansäureoctadecylester |
| 20,0 | Glycerintristearat |
| 46,5 | Paraffinöl |

Beispiel 2

Sonnenschutzmilch

| | | |
|---|---|---|
| a) | 2,0 | Fettalkohol 6fach ethoxyliert |
| | 2,0 | Fettalkohol 25fach ethoxyliert |
| | 5,0 | Äthylhexansäureoctadecylester |
| | 5,5 | Glycerintristearat |
| | 13,0 | Paraffinöl |
| | 4,0 | Stearinsäure |
| | 5,2 | 4-Butoxyzimtsäuremethylester |
| | 0,8 | Cetylalkohol |
| | 0,2 | p-Hydroxybenzoesäuremethylester |
| b) | 2,0 | Triäthanolamin |
| | 60,0 | Wasser |
| c) | 0,3 | Parfümöl |

Man erwärmt die Phasen a und b auf 70°C und rührt Phase b in Phase a ein. Wenn das gerührte Gemisch auf 40°C abgekühlt ist, gibt man die Phase c zu und rührt weiter bis Raumtemperatur erreicht wird.

Beispiel 3

Sonnenschutzcreme, weich

a) 1,0 Stearinsäure 9fach ethoxyliert
1,0 Fettalkohol 6fach ethoxyliert
1,0 Fettalkohol 25fach ethoxyliert
10,0 Stearinsäure
2,0 Vaseline
11,0 Paraffinöl
4,0 Bienenwachs hell
0,5 Siliconöl
2,0 Äthylhexansäureoctadecylester
5,5 p-tert.-Butoxyzimtsäuremethylester

b) 4,0 Propylenglykol
0,2 p-Hydroxybenzoesäuremethylester
0,5 Triäthanolamin
57,3 Wasser

Die Phasen a und b werden auf 75°C erwärmt und Phase b in Phase a eingerührt. Man rührt bis zum Abkühlen auf 40°C weiter, gibt nach Belieben Parfümöl zu und rührt bis zum Erreichen der Raumtemperatur nach.

D

## Patentansprüche

1. Verwendung von Verbindungen der allgemeinen Formel I

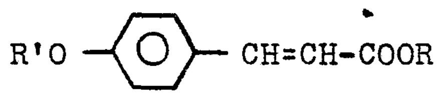 (I)

in der R' einen geradkettigen oder verzweigten Alkylrest mit 3 bis 15 Kohlenstoffatomen, einen Benzyl- oder Phenylrest und R einen geradkettigen oder verzweigten Alkylrest mit 1 bis 15 Kohlenstoffatomen oder den ß-Alkoxyäthan-, 3-Alkoxypropan-, einen Cyclohexyl- oder Benzylrest bedeuten

als Lichtschutzmittel oder UV-Stabilisator.

2. Verwendung von Verbindungen der Formel I als Lichtschutzmittel für die menschliche Haut.

BAD ORIGINAL